Europäisches Patentamt

European Patent Office

Office européen des brevets

(11) Veröffentlichungsnummer: **0 164 545**
A2

# (12) EUROPÄISCHE PATENTANMELDUNG

(21) Anmeldenummer: 85105187.0

(22) Anmeldetag: 29.04.85

(51) Int. Cl.⁴: **C 12 N 1/26**
C 12 N 11/00, C 12 M 1/08

(30) Priorität: 11.05.84 DE 3417443

(43) Veröffentlichungstag der Anmeldung:
18.12.85 Patentblatt 85/51

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Voelskow, Hartmut, Dr.
Akazienstrasse 22
D-6234 Hattersheim am Main(DE)

(72) Erfinder: Lingens, Franz, Prof. dr.
Im Asemwald 32/4
D-7000 Stuttgart 70(DE)

(72) Erfinder: Bauer, Alexander, Dr.
Lerchenweg 3
D-6233 Kelkheim (Taunus)(DE)

(72) Erfinder: Keller, Reinhold, Dr.
Wiesenweg 5
D-6232 Bad Soden am Taunus(DE)

(72) Erfinder: Faust, Uwe, Dr.
Kelkheimer Strasse 27
D-6233 Kelkheim (Taunus)(DE)

(54) Bakterienkulturen, Verfahren zu ihrer Herstellung und ihre Verwendung.

(57) Der biologische Abbau schwer abbaubarer Aromaten gelingt mit Bakterienkulturen, die durch aerobes Züchten in einem Mineralmedium mit schwer abbaubaren Aromaten als einzige C-Quelle erhalten werden. Als Ausgangsmaterial dienen vorteilhaft Proben, die am natürlichen Standort bereits schwer abbaubare Komponenten enthalten.

EP 0 164 545 A2

0164545

HOECHST AKTIENGESELLSCHAFT       HOE 84/F 103      Dr.KL/St

Bakterienkulturen, Verfahren zu ihrer Herstellung und
ihre Verwendung

Die Erfindung betrifft Bakterienkulturen, insbesondere
Bakterien-Mischkulturen, die befähigt sind, biologisch
schwer abbaubare Aromaten abzubauen, Kultivierungsverfahren zur Gewinnung und Stabilisierung solcher Kulturen sowie ihre Verwendung zur Reinigung von Abwässern, die die
genannten aromatischen Verbindungen enthalten.

Biologische Verfahren werden weltweit zum Abbau kommunaler
und industrieller Abwässer eingesetzt. In Anbetracht der
zu behandelnden Volumenströme, der Schwankungen der Abwasserzusammensetzung und jahreszeitlicher Einflüsse auf
Abwasseranlagen wurden bisher vorrangig technische Verbesserungen wie Optimierung des Sauerstofftransfers, der
Mischgüte, der Korrosionsfestigkeit der Abwasserbecken,
der Schlammabtrennung, der Verringerung des Platzbedarfs
und der Bauform der Behälter angestrebt. Für spezielle
Abwässer wurden in letzter Zeit durch Fermentation mikrobiologische Starterkulturen hergestellt, mit denen bestehende Abwasseranlagen beschickt werden können. Mit den
bekannten Verfahren und Kulturen wird großtechnisch ein
weitestgehender aerober oder anaerober Abbau erreicht.
Wenn die Abwasserströme allerdings schwer abbaubare organische Substanzen wie chlorierte Aromaten enthalten, verbleibt im geklärten Wasser ein Rest-CSB (CSB = Chemischer
Sauerstoffbedarf), der als BSB (Biologischer Sauerstoffbedarf) nicht feststellbar ist. Solche Verbindungen, deren
besondere Beachtung bereits in der EG-Richtlinie vom 4.5.76
gefordert wurde, kommen nur in sehr geringen Konzentrationen
in industriellen Abwässern vor, so daß eine chemische oder
adsorptive Abtrennung nicht wirtschaftlich ist.

Es bestand deshalb die Aufgabe, schwer abbaubare organische Substanzen, wie sie nach weitestgehender innerbetrieblicher Abtrennung in Abwasserströme abgegeben werden, biologisch zu entfernen.

Aus der europäischen Patentanmeldung mit der Veröffentlichungsnummer 99 020 ist es bekannt, daß mehrfach substituierte Kohlenwasserstoffe mit nicht identischen Substituenten durch Mikroorganismen abgebaut werden können, die an positive Ladungen tragende Trägermaterialien anhaften. Diese Mikroorganismen können mit den mehrfach substituierten aromatischen Kohlenwasserstoffen als einzige Kohlenstoffquelle vermehrt werden, wobei zunächst die Mikroorganismen in verschiedenen Einzelkulturen adaptiert werden, die als einzige Kohlenstoffquelle Aromaten enthalten, die nur einen oder weniger Substituenten als die abzubauende Substanz tragen. Erst in einer späteren Stufe werden dann die vereinigten Ausgangskulturen an die abzubauenden Substanzen adaptiert. Dieses Verfahren ist insofern ziemlich aufwendig, als eine Vielzahl von Kulturen isoliert, selektiert und adaptiert werden, die nur die niedriger substituierten Substanzen abbauen können.

Es wurde nun gefunden, daß spezielle Bakterienkulturen in der Lage sind, auch schwer abbaubare, beispielsweise chlor- und stickstoffhaltige, Aromaten aus Abwässern zu eliminieren. Die erfindungsgemäßen Bakterienkulturen sind erhältlich durch aerobes Züchten von Bakterien aus Mikroorganismen enthaltenden Proben in einem Temperaturbereich von 15 bis 40°C, bevorzugt 20 bis 35°C, insbesondere 22 bis 30°C, in einem Mineralmedium, das als einzige Kohlenstoffquelle ein schwer abbaubares Isomeres oder mehrere schwer abbaubare Isomere aus der Gruppe der Toluidine, Chlor-methyl-aniline, der Chlor-nitro-aniline und der Chlor-nitro-benzole enthält, unter mehrfachem Überimpfen der auf eine Zelldichte $OD_{580} >$ 0,5 wachsenden Kulturen, die den Aromaten abbauen, in jeweils frisches Medium.

Der Begriff "als einzige Kohlenstoffquelle" soll hierbei nicht ausschließen, daß den Kulturen leichter abbaubare Verbindungen, insbesondere niedriger substituierte Aromaten (bei chlorhaltigen Aromaten beispielsweise die entsprechenden chlorfreien), zugesetzt werden können. Ein solcher Zusatz ist in vielen Fällen sogar vorteilhaft, weil dadurch die Kulturen schneller wachsen, ohne aber ihre guten Eigenschaften zu verlieren.

Im Gegensatz zu dem vorstehend beschriebenen bekannten Verfahren werden also erfindungsgemäß durch unmittelbare Adaption an die abzubauenden höher substituierten Aromaten Arbeitsschritte eingespart, die lediglich Kulturen liefern, die nur niedriger substituierte Aromaten abbauen können. Hierdurch wird auch die Erfolgswahrscheinlichkeit wesentlich erhöht. So wurde gefunden, daß im Falle des Chlor-methyl-anilins Einzelkulturen, welche auf Anilin, Toluol und Chlorbenzol wuchsen, auch nach Mischung nicht zum Erfolg führten, während überraschenderweise gleich mehrere Ansätze mit Chlor-methyl-anilin-haltigen Medien zum Auffinden von geeigneten Kulturen führten.

Weitere vorteilhafte Ausführungsformen der Herstellung und Verwendung der erfindungsgemäßen Kulturen werden im folgenden näher erläutert:

Geeignete Mikroorganismen enthaltende Proben sind insbesondere solche, die am natürlichen Standort bereits schwer abbaubare Komponenten enthalten wie Koniferen-Walderde mit teilweise verrotteten Nadeln, alte Koniferen-Rinden und -Holzspäne, ölverschmutzte Erde, Klärschlamm, Boden vom Rand asphaltierter Straßen, Meerwasser und verschlammter Sand aus verschmutzten Seeküstengebieten. Aber auch Pferdedung, Material aus gärenden Misthaufen, Erde aus Gärten oder verschiedener Ackerböden oder Schlamm von Flußrandzonen in 5 bis 10 cm Tiefe kann zur Anreicherung solcher Mikroorganismen verwendet werden.

Als besonders gut geeignet haben sich die folgenden zwei Methoden für die Anreicherung, Selektion und Adaption der mehrfach substituierte Aromaten abbauenden Mikroorganismen erwiesen:

1. Flüssigkultur zur Simulation flacher aerober Gewässer:

Kulturgefäße mit 500 ml Füllmenge eines geeigneten Mineralmediums ohne Kohlenstoffquelle werden mit 100 bis 500 ppm einer oder mehrerer der mehrfach substituierten aromatischen Verbindungen und mit 2 bis 200 g, bevorzugt 30 bis 80 g, Probenmaterial versetzt. Die Kultur wird mit einem Rührer leicht gerührt oder auf einer Schüttelmaschine nur sehr langsam (beispielsweise mit 40 bis 60 Upm bei 2,5 cm Schütteldurchmesser) geschüttelt. Eine stärkere Belüftung ist für die Anreicherung der gewünschten Bakterienkulturen weder erforderlich noch vorteilhaft, da sich andere Bakterien bevorzugt vermehren könnten, die im Probenmaterial noch genügend C-Substrate finden.

2. Erdkultur zur Simulation der oberen Bodenschichten an natürlichen Standorten:

Die Erdprobe wird, sofern genügend Probenmaterial vorhanden ist, unmittelbar in kleine Ton-Blumentöpfe gefüllt bzw. zunächst mit Gartenerde, Ackererde, Tonsplittern oder grobem Sand vermischt und diese Mischung dann in die Tontöpfe gefüllt. Die Töpfe werden mit einem Mineralmedium begossen, welches den bzw. die mehrfach substituierten oben genannten Aromaten enthält, wobei die Probe regelmäßig feucht gehalten wird.

Die Kulturen nach den genannten beiden Verfahren werden bei Temperaturen von etwa 15 bis 40°C, vorzugsweise 20 bis 35°C, insbesondere 22 bis 30°C, bebrütet, bis ein Abbau der eingesetzten Substanz feststellbar ist. Das kann innerhalb von 14 Tagen der Fall sein, beispielsweise bei Chlornitrobenzolen, oder auch einige Monate dauern. Aus der Flüssigkultur wird allerdings nach 8 bis 12 Wochen in jedem Fall eine neue Kultur mit 5 bis 10 % Inokulum angesetzt, bis entweder Abbau feststellbar ist oder nach mehreren Passagen im Falle eines Mißerfolgs alle Bakterien herausverdünnt sind.

Die beiden genannten Verfahren können zur Erhöhung der Erfolgschance auch parallel zueinander angesetzt werden. Kulturen werden daraus nach gängigen mikrobiologischen Techniken isoliert. Sofern hierbei eine reine Kultur gewonnen wird, die die gleiche Abbauleistung zeigt, wird sie als Reinkultur weiterverwendet. Sofern die Abbauleistungen der isolierten Reinkulturen unvollständig sind oder die Aktivität schwächer ist als in der Anreicherungskultur, werden die zum Abbau notwendigen Stämme in Mischung belassen und als Mischkultur weiterverwendet. Da die verschiedenen Bakterien sich in der Abbauaktivität gegenseitig ergänzen, bilden sie unter den selektiven Bedingungen der abzubauenden Substanz als alleinige C-Quelle stabile Mischkulturen.

Die Kulturen, die die Testsubstanzen abbauen, bleiben auch nach regelmäßigem Überimpfen in gleiche Bedingungen über ein Jahr stabil und können durch Selektion auf steigende Konzentrationen verbessert werden, das heißt die Abbaugeschwindigkeit und das Wachstum werden erhöht.

Im Laufe von 18 Monaten konnten für alle getesteten Aromaten abbauende Kulturen gefunden werden. Hierdurch ist gezeigt, daß erfindungsgemäß mit hoher Erfolgswahrscheinlichkeit Kulturen gewonnen werden können, die schwer abbaubare Substanzen biologisch abbauen.

Überraschenderweise wurde gefunden, daß in der Regel die erfindungsgemäßen Mischkulturen sehr viel leistungsfähiger und stabiler sind als daraus isolierte Reinkulturen, die nur Abbauraten von 30 bis 70 % erreichten, ausgenommen in den Fällen, in denen von vornherein eine reine Kultur als Abbauer isoliert wurde.

Die Anreicherungskulturen werden zweckmäßig in Laborfermentern von 1 bis 1,5 l Inhalt unter leichter Belüftung und Rühren gezüchtet. Gut wachsende Kulturen werden in 20 l Schlaufenreaktoren, die nach dem Airlift-Prinzip betrieben werden, kultiviert. Diesen Ansätzen werden zur besseren C-Versorgung neben den schwer abbaubaren Verbindungen (150 ppm) auch leichter abbaubare, z.B. niedriger substituierte Analoga (500 ppm) zugegeben und nach Verbrauch ergänzt.

Ein weiterer besonderer Vorteil des erfindungsgemäßen Verfahrens liegt darin, daß eine wesentlich verbesserte Zellvermehrung durch den Zusatz relativ leicht abbaubarer C-Substrate in der Zellanzucht erreicht werden kann. Geeignet sind Glycerin, Mannit, Sorbit, Paraffine, aliphatische Alkohole und Carbonsäuren mit jeweils bis zu 18 Kohlenstoffatomen, aromatische Verbindungen mit mindestens einer Hydroxygruppe oder einer oder zwei Carboxygruppen am aromatischen Ring sowie gegebenenfalls die genannten niedriger substituierten Analoga zu den abzubauenden Aromaten, beispielsweise die chlorfreien analogen Verbindungen. Besonders bevorzugt ist ein Zusatz von Glycerin, Mannit, Sorbit oder niederen Alkanolen wie Methanol und insbesondere Ethanol. Vorversuche ergeben jeweils das am besten geeignete Substrat. Die Menge dieser Zusätze liegt bevorzugt bei 1 bis 5 g pro Liter Medium. Sofern der Zusatz ein Lösevermögen für die abzubauenden Substrate darstellt, wie beispielsweise Ethanol, so werden die abzubauenden Substanzen vorteilhaft in Form der entsprechenden Lösungen zugeführt, was zu einer besseren Verteilung im Medium führt.

Der durch den Zusatz dieser relativ leicht abbaubaren C-Substrate erreichte Effekt ist überraschend, da Vergleichsversuche zeigen, daß Substrate, die über Glucose-6-phosphat metabolisiert werden, zu einer Verminderung der Abbaufähigkeit für substituierte Aromaten führen. Dies hat bei einer kontinuierlichen Zellanzucht eine Ausspülung der abbauaktiven Kultur zur Folge.

Die Verweilzeit der substituierte Aromaten abbauenden Kulturen in der kontinuierlichen Zellanzucht beträgt etwa 20 Stunden bis 50 Stunden. Bei Zusatz der genannten leichter abbaubaren C-Substrate kann die Verweilzeit auf 5 bis 10 Stunden verkürzt werden, wobei gleichzeitig eine bis zum 5-fachen erhöhte Zelldichte erreicht wird (Extinktionswerte bei 580 nm im Bereich von 2 bis 5 gegenüber 0,4 bis 1 ohne diese Zusätze).

In einer vorteilhaften Ausgestaltung der Erfindung wird die Bakterienkultur in Form fixierter Zellen eingesetzt. Immobilisierte Biokatalysatoren, insbesondere in Form von Perlen, sind bekannt und beispielsweise in den deutschen Offenlegungsschriften 2 343 633 (US-Patentschrift 4 070 348) und 2 805 607 beschrieben. Ein besonders vorteilhaftes Verfahren ist in der deutschen Offenlegungsschrift 3 237 341 beschrieben. Diese fixierten Zellen erlauben bei weitestgehender Erhaltung ihrer biologischen Aktivität eine erheblich verbesserte Durchmischung mit den zu behandelnden Abwässern und eine wesentlich erleichterte Abtrennung und Rückführung des Biokatalysators aus den behandelten Abwässern.

Für die Immobilisierung der Kulturen wurde das Verfahren nach der deutschen Offenlegungsschrift 3 237 341 bevorzugt, nach dem perlförmige Biokatalysatoren durch radikalische Polymerisation von Acrylamid und Methylen-bis-acrylamid in wäßriger Suspension unter Zusatz einer hochfluorierten Kohlenstoffverbindung erhalten werden. Hierfür werden die Mikroorganismen als Feucht-Zellmasse eingesetzt, zweckmäßig als abzentrifugierte und gewaschene Zellmasse, die in physiologischer Kochsalzlösung aufgenommen wird. Hierzu werden die Monomeren Acrylamid und Methylen-bis-acrylamid gegeben, unter kräftigem Rühren in der hochfluorierten Kohlenstoffverbindung suspendiert und die Polymerisation gestartet. Die erhaltene Perlform des Biokatalysators ergibt sehr gute Raum-Zeit-Ausbeuten und bedingt eine hohe Stabilität und Aktivität. Es ist somit ein universeller Einsatz in den verschiedensten Reaktortypen möglich.

Bei der erfindungsgemäßen Anwendung der Bakterienkulturen muß den großen zu verarbeitenden Volumina und den dadurch bedingten Durchmischungsproblemen Rechnung getragen werden.

So ist - selbst beim Einsatz der bevorzugten trägerfixierten Kulturen - darauf zu achten, daß hohe lokale Scherkräfte und das Kompaktieren des beladenen Trägermaterials vermieden werden. Darüber hinaus ist sicherzustellen, daß die Konzentrationsgradienten der abzubauenden Stoffe und ihrer teilweise hochreaktiven Abbaustufen gering gehalten werden, wobei gleichzeitig für gute Durchmischung des gesamten Biokatalysatormaterials mit dem umzusetzenden Medium gesorgt werden muß. Außerdem müssen die Steuergrößen Sauerstoffgehalt, pH-Wert, Ionenkonzentration, Substratkonzentration, Temperatur und Zwischenproduktkonzentrationen jeder Zeit an jeder Stelle des Reaktionsgemisches meßbar sein. Weiterhin sind die immobilisierten Kulturen im Reaktionssystem zurückzuhalten, um eine hohe Abbaugeschwindigkeit bei geringer Wachstumsgeschwindigkeit zu ermöglichen.

Diese Anforderungen erfüllt in besonders zweckmäßiger Weise das in der (nicht vorveröffentlichten) deutschen Offenlegungsschrift 3 247 214 vorgeschlagene Verfahren zur Durchführung enzymatischer Reaktionen mit immobilisierten Biokatalysatoren, das dadurch gekennzeichnet ist, daß die Reaktion in einem Reaktor nach dem Schlaufenprinzip erfolgt. Hierbei wird das zu behandelnde Abwasser kontinuierlich einem mit der immobilisierten Kultur gefüllten, begasten Schlaufenreaktor in der Form zugeführt, daß die freie Konzentration an Substrat und Zwischenprodukten gegen Null gehalten wird. Hierzu werden die Durchflußzeit und Substratkonzentration im Zulauf der erreichbaren Abbaurate angepaßt. Bei hoher Substrateingangskonzentration kann eine Vorverdünnung mit bereits abgebautem Medium durch dessen partielle Rückführung erreicht werden. Während des Verfahrens werden im Bioreaktor der pH-Wert, die Temperatur, die Konzentration an Sauerstoff, Mineralien und weiteren für eine optimale Umsetzung notwendigen Nährstoffen eingestellt und konstant gehalten. Die Umwälzung und Mischintensität sowie die Entfernung gasförmiger Produkte wie Kohlendioxid erfolgt durch Begasung des Reaktors am Boden in den Aufstiegsteil des Schlaufenreaktors hinein. Die Begasungsrate wird hierbei so eingestellt, daß eine schonende, aber gleichmäßige Durchmischung und Umwälzung erfolgt.

Die Rückhaltung des Biokatalysators erfolgt in einer dem Ablauf vorgeschalteten Beruhigungszone, in der eine Sedimentation erfolgt. Das Sediment wird in den Reaktionsraum zurückgefördert, während der klare geklärte Überstand als freier Überlauf in die dafür vorgesehene Weiterverarbeitungsstufe (Vorfluter) abfließt. Die Sedimentationsstufe kann mit der Reaktionsstufe konstruktiv kombiniert werden, wie es beispielsweise in den europäischen Patentschriften 3 547 und 3 548 (US-Patentschrift 4 251 371) beschrieben ist, oder aber getrennt angeordnet sein, wie es beispielsweise aus der deutschen Auslegeschrift 2 938 339 bzw. US-Patentschrift 4 346 113 hervorgeht.

In den folgenden Beispielen wird die Erfindung näher erläutert.

Beispiel 1:
a) Isolierung einer Kultur

In vier gerührte Gefäße mit jeweils 500 ml Mineralmedium wurden je 10 g einer Mischung aus teilweise verrotteten Fichtennadeln, Fichten- und Eichenholzspänen sowie -rindenstücken gegeben. In zwei Gefäßen enthielt das Medium zusätzlich 100 ppm 3-Chlor-2-methyl-anilin, in den beiden anderen Gefäßen 400 ppm 2-Methylanilin. Die Mischungen wurden 4 Wochen lang bei Zimmertemperatur ohne zusätzliche Belüftung gerührt. Anschließend wurden je 5 ml entnommen und wiederum zu je 500 ml neuen gleichen Ansätzen gegeben. Dieser Vorgang wurde noch ein drittes Mal wiederholt und anschließend weitere drei Monate lang in Abständen von jeweils 14 Tagen. Danach war die ursprüngliche Erdprobe restlos herausverdünnt; dennoch zeigte sich in den Kolben eine leichte Trübung durch bakterielles Wachstum.

Ein Kontrollversuch zeigte, daß die C-Quellen nach 7 Tagen zu 100 % abgebaut wurden. Ein Blindansatz ohne Bakterienbeimpfung zeigte nur ca. 5 % Verdunstungsverlust.

Kreuzversuche mit den isolierten Kulturen zeigten, daß die 2-Methylanilin abbauenden Organismen auch 3-Chlor-2-methylanilin abbauten. Die Kulturen in den vier Anreicherungsgefäßen verhielten sich weitgehend gleich und wurden vereinigt und als Impfmaterial für die weiteren Versuche verwendet.

b) Kulturverbesserung

5 ml der Kultur wurden in 500 ml Mineralmedium überimpft, das 500 ppm 2-Methyl-anilin und 150 ppm 3-Chlor-2-methylanilin enthielt. Nach einer Wachstumszeit von 5 Tagen wurde die gleiche Menge der Aromaten zugegeben. Nach weiteren 5 Tagen erreichte die Kultur eine $OD_{580} > 1,5$ und wurde hierauf in einen 20 l-Airlift-Fermenter mit dem gleichen Medium überführt. Aufgrund der besseren Kulturbedingungen war im Airlift-Fermenter das Wachstum schneller, so daß nach 3 und 5 Tagen die Aromaten (jeweils in der gleichen Menge) nachdosiert werden konnten. Die Kultur erreichte eine Dichte $OD_{580} > 2,6$ und baute die Testsubstanzen in 7 Tagen vollständig ab.

Die Zellmasse der Kultur wurde durch Zentrifugieren abgetrennt und für weitere Versuche verwendet.

c) Fixierung der Zellen und Einsatz im Schlaufenreaktor

Die abzentrifugierte feuchte Zellmasse kann nach Beispiel 1 der deutschen Offenlegungsschrift 3 237 341 in einen perlförmigen Biokatalysator überführt werden, der gemäß der deutschen Offenlegungsschrift 3 247 214 in einem Schlaufenreaktor eingesetzt werden kann, in dem Abwässer mit schwer abbaubaren Aromaten biologisch abgebaut werden sollen.

Beispiel 2

In Blumentöpfe wurden Mischungen aus 150 g Ackererde und 0,25 g o-Toluidin gefüllt und bei 30°C feucht gehalten. Nach Abbau des o-Toluidins (dünnschichtchromatographische Bestimmung) wurde etwa 1 g der Erde in ein Mineralmedium mit 0,4 g o-Toluidin/l überführt. Nach komplettem Abbau wurde erneut überimpft und nach mehreren Überimpfungen eine Bakterien-Reinkultur erhalten, die als Rhodococcus rhodochrous identifiziert wurde.

Die so erhaltene Kultur baute 400 mg/l o-Toluidin nach 3 Tagen komplett ab. Wachstum erfolgte ebenfalls mit m-Toluidin und mit Anilin als jeweils einziger Kohlenstoffquelle. Die mit o-Toluidin als einziger C-Quelle erhaltenen Kulturen bauten neben m-Toluidin und Anilin auch 6-Chlor-2-methylanilin ab.

Beispiel 3

Gartenerde-Proben wurden in ein Mineralmedium mit 0,2 g/l p-Toluidin überführt und bei 30°C bebrütet. Nach mehrfachem Überimpfen in frisches Medium wurde eine Bakterien-Reinkultur erhalten, die als Pseudomonas testosteroni identifiziert wurde. Die erhaltenen Kulturen bauten 0,3 g/l p-Toluidin im Lauf von 8 Stunden ab.

PATENTANSPRÜCHE:

1. Bakterienkulturen, erhältlich durch aerobes Züchten von Bakterien aus Mikroorganismen enthaltenden Proben in einem Temperaturbereich von 15 bis 40°C in einem Mineralmedium, das als einzige Kohlenstoffquelle ein schwer abbaubares Isomeres oder mehrere schwer abbaubare Isomere aus der Gruppe der Toluidine, der Chlor-methyl-aniline, der Chlor-nitro-aniline und der Chlor-nitro-benzole enthält, unter mehrfachem Überimpfen der auf eine Zelldichte $OD_{580}$ > 0,5 wachsenden Kulturen in jeweils frisches Medium.

2. Verfahren zur Herstellung von Bakterienkulturen durch aerobes Züchten in einem Temperaturbereich von 15 bis 40°C in einem Mineralmedium, das als einzige Kohlenstoffquelle einen Aromaten enthält, dadurch gekennzeichnet, daß als Aromat ein schwer abbaubares Isomeres oder mehrere schwer abbaubare Isomere aus der Gruppe der Toluidine, der Chlor-methyl-aniline, der Chlor-nitro-aniline und der Chlor-nitro-benzole eingesetzt werden und Adaption an den genannten Aromaten erfolgt.

3. Verfahren nach Anspruch 2, dadurch gekennzeichnet, daß das Züchten bei 20 bis 35°C erfolgt.

4. Verfahren nach Anspruch 2 oder 3, dadurch gekennzeichnet, daß das Züchten bei 22 bis 30°C erfolgt.

5. Verfahren nach einem oder mehreren der Ansprüche 2 bis 4, dadurch gekennzeichnet, daß dem Medium als zusätzliche C-Quelle eine leichter abbaubare Verbindung zugesetzt wird.

6. Verwendung der Bakterienkulturen nach Anspruch 1 bzw. der nach einem oder mehreren der Ansprüche 2 bis 5 erhaltenen Kulturen zum Abbau schwer abbaubarer Aromaten.

7. Verwendung der Bakterienkulturen gemäß Anspruch 6 in Form fixierter Zellen.

8. Verwendung der Bakterienkulturen gemäß Anspruch 6 oder 7 in einem Schlaufenreaktor.

PATENTANSPRÜCHE für Österreich:

1. Verfahren zur Herstellung von Bakterienkulturen durch aerobes Züchten von Bakterien aus Mikroorganismen enthaltenden Proben in einem Temperaturbereich von 15 bis 40°C in einem Mineralmedium, das als einzige Kohlenstoffquelle einen Aromaten enthält, dadurch gekennzeichnet, daß als Aromat ein schwer abbaubares Isomeres oder mehrere schwer abbaubare Isomere aus der Gruppe der Toluidine, der Chlor-methyl-aniline, der Chlor-nitro-aniline und der Chlor-nitro-benzole eingesetzt werden und die auf eine Zelldichte $OD_{580} > 0,5$ wachsenden Kulturen mehrfach in jeweils frisches Medium überimpft werden.

2. Verfahren nach Anspruch 1, dadurch gekennzeichnet, daß eine direkte Adaption an den genannten Aromaten erfolgt.

3. Verfahren nach Anspruch 1 oder 2, dadurch gekennzeichnet, daß das Züchten bei 20 bis 35°C erfolgt.

4. Verfahren nach Anspruch 1 bis 3, dadurch gekennzeichnet, daß das Züchten bei 22 bis 30°C erfolgt.

5. Verfahren nach einem oder mehreren der Ansprüche 1 bis 4, dadurch gekennzeichnet, daß dem Medium als zusätzliche C-Quelle eine leichter abbaubare Verbindung zugesetzt wird.

6. Verwendung der nach einem oder mehreren der Ansprüche 1 bis 5 erhaltenen Mischkulturen zum Abbau schwer abbaubarer Aromaten.

**0164545**

7. Verwendung der Bakterienkulturen gemäß Anspruch 6 in Form fixierter Zellen.

8. Verwendung der Bakterienkulturen gemäß Anspruch 6 oder 7 in einem Schlaufenreaktor.